# EUROPEAN PATENT APPLICATION

(11) **EP 1 639 979 A2**
(43) Date of publication of application: **29.03.2006**
(21) Application number: 05255738.6
(22) Date of filing: 15.09.2005
(51) Int. Cl.: A61F 13/551

(54) **Rolled disposable absorbent articles**

(30) Priority: 16.09.2004 US 942661
(71) Applicant: McNeil-PPC, Inc., Skillman, NJ 08558 (US)
(72) Inventor: Bohlen, Adalberto, Rua Ricardo de Brito Campoy, 73, Sao Paulo CEP 122544-492 (BR); Moscherosch, H. Michael, Doylestown, PA 19801 (US); Pereira, Joao B., Manoel Martins Junior Str. 790, Sao Paulo 12242-810 (BR); Sturgeon, Jennifer L., Long Valley, NJ 07853 (US)
(74) Representative: Mercer, Christopher Paul

(57) **Abstract**

An individually rolled disposable absorbent article having a disposable absorbent article wherein the disposable absorbent article is rolled from one end portion to the opposed end portion to form the rolled disposable absorbent article and upon unrolling the rolled disposable absorbent article, the absorbent article maintains substantially no memory of its prior rolled configuration. Packaging containing such individually rolled disposable absorbent article and methods of making such individually rolled disposable absorbent articles are presented.

## Description

This invention provides a package for sanitary absorbent articles and a method of packaging the absorbent articles. The present invention also relates to absorbent articles that are individually packed in a rolled configuration, thereby occupying little space. The rolled absorbent articles are further contained in an outer wrap, which helps maintain the rolled shape and protect the absorbent article from contamination. Also disclosed in this invention is the process of rolling the absorbent product, wrapping the rolled absorbent product to form a final product, and placing the final product in secondary packaging for shipping.

### BACKGROUND OF THE INVENTION

Disposable absorbent articles, such as, pantiliners, sanitary napkins, interlabial devices, adult incontinence devices, bandages, breast pads, and diapers are well known in the art. These articles typically have a fluid permeable body-facing side and fluid impermeable garment facing side and may include an absorbent core for retaining fluids therebetween. Such absorbent structures have traditionally been made from readily available and relatively inexpensive materials, such as, cotton fibers, wood pulp fluff, cellulosic tissue, or wadding, or other absorbent materials. These materials have provided satisfactory absorbency of fluids both in terms of absorbency rate and overall absorbent capacity.

Conventionally, absorbent articles, especially sanitary napkins and pantiliners, are commercially available in two different package types.

In one type, a plurality of sanitary articles are positioned within a container such that they are stacked, usually side-by-side. In this type of package, the consumer opens the container, takes out an sanitary article and uses it. The drawbacks to this package type is twofold: the sanitary article has no other protection against contamination, for example, if the user places the article in a handbag for use later and secondly, the container is now open and the remaining articles may be contaminated over time. Another type of commercial package available on the market is the individual fold and wrap (IFW). In this package type, each sanitary article is enclosed within a plastic-type package. While this package type may have the convenience of being safe and discreet for use in a handbag, often the type of folding and wrapping suffers from problems. For example, the individual IFW package typically has one side that is open or incompletely sealed, which would allow contaminates such as dust and dirt to enter the interior of the package. Additionally, the sanitary article, in order to be packed, is folded once or twice, creating at least one transverse fold or a flow channel.

Examples of packaging for sanitary napkins can be found U.S. Pat. Nos. 4,505,704 ("'704"); 6,254,582 ("'582"); 4,598,528 ("'528"); and 4,564,108 ("'108"). '704 discloses a continuous strip of napkin material configured in a single roll and mounted in a dispenser. The dispenser has a cutting edge to allow for a transverse tear to separate a segment of desired length by the user. '582 discloses a supply of absorbent panty liners formed by a contiguous longitudinal array of pad segments forming a strip. Each pad segment is shorter than a standard panty liner pad so that by tearing off two, three, four or five segments in one piece, the user can create a panty liner having a length to suit her needs. '528 discloses a series of diapers connected by easily severably areas. These diapers are rolled and may be contained in a dispensing container that allows them to be withdrawn a single diaper at a time. '108 discloses a plurality of napkins having their adhering side releasably fixed to a plastic film. The film may be folded such that the napkins are placed on top of each other forming one or more piles enclosed by the film.

Other types of packaging are known in the art. For example, U.S. Pat. No 5,964,741 ("741") discloses a combination of a vaginal insert and an external absorbent article and includes a pouch. As the external absorbent article is laterally or longitudinally rolled about the vaginal insert, the diameter of the rolled absorbent article must be greater than the vaginal insert. The resulting package may not be discreet enough to be carried in a small purse or pants pocket.

While all of the described patents describe ways to package sanitary articles, none disclose a form that can be easily and discreetly carried in a users purse or pocket. What is needed therefore, is a package that protects the sanitary article from contamination and contains the sanitary article discreetly. Additionally, a method of making such a sanitary article and package is also needed.

Unless specifically defined herein, all technical and scientific terms used herein have the same meaning as commonly understood by one of ordinary skill in the art to which this invention belongs. Although methods and materials similar or equivalent to those described herein can be used in the practice or manufacturing of the present invention, suitable methods and materials are described below. Any publications, patent applications, patents and other documents mentioned herein are incorporated by reference in their entirety. In case of conflict between any document mentioned herein and the instant specification, including definitions, the instant specification will control. In addition, the materials, methods, and examples provided herein are illustrative only and are not intended to be limiting.

Other features and advantages of the invention, e.g., individually rolled disposable absorbent article, will be apparent from the following description and from the claims.

### SUMMARY OF THE INVENTION

An individually rolled disposable absorbent article comprising, consisting of, and/or consisting essentially of a disposable absorbent article comprising a silhouette comprising two opposed end portions and two opposed longitudinal edges connecting the two opposed end portions and b) a plurality of layers comprising a cover and a backsheet, wherein the disposable absorbent article is rolled from one end portion to the opposed end portion to form the rolled disposable absorbent article and upon unrolling the rolled disposable absorbent article, the absorbent article maintains substantially no memory of its prior rolled configuration.

An individually rolled disposable article of any of the previous claims, wherein the rolled disposable article is maintained in a rolled position using a device selected from the group consisting of a wrapper, a ring, a hook and loop, an adhesive, and mixtures thereof.

An individually rolled disposable absorbent article comprising, consisting of, and/or consisting essentially of a disposable absorbent article consisting essentially of a) a silhouette comprising two opposed end portions and two opposed longitudinal edges connecting the two opposed end portions and b) a plurality of layers comprising a spunlace cover and a microporous film backsheet, wherein the disposable absorbent article is rolled from one end portion to the opposed end portion to form the rolled disposable absorbent article and upon unrolling the rolled disposable absorbent article, the absorbent article maintains substantially no memory of its prior rolled configuration.

A package for holding a plurality of individually rolled disposable absorbent articles comprising, consisting of, and/or consisting essentially of a container having a top, base and at least one side connecting the top to the base, means for removing an individually rolled disposable absorbent article from the container, and a plurality of individually rolled disposable absorbent articles.

A method of providing a package containing at least one individually rolled disposable absorbent articles, the method comprising, consisting of, and/or consisting essentially of: a) providing a cover material, (b) providing a backsheet, (c) securing the cover to the backsheet to provide a disposable absorbent article, (d) rolling the disposable absorbent article to form an individually rolled disposable absorbent article, (e) applying means to maintain the individually rolled disposable absorbent article in a rolled configuration, and (f) placing the individually rolled disposable absorbent article in a package.

### BRIEF DESCRIPTION OF THE DRAWINGS

FIG. 1 is a top plan view of an absorbent article according to the invention;
FIG. 2 is a cross-sectional view taken across line A-A of the embodiment shown in FIG. 1;
FIG. 3 is a cross-sectional view of an alternate embodiment of the invention containing an absorbent core;
FIG. 4 is a top plan view of one embodiment of a rolled disposable absorbent article according to the invention;
FIG. 5 is an elevational view of a one embodiment of a rolled disposable absorbent article contained in an overwrap;
FIG. 6 is an elevational view of an alternate embodiment of a rolled disposable absorbent article surrounded by a ring;
FIG. 7 is a perspective view of one embodiment of a container for holding a plurality of rolled disposable absorbent articles;
FIG. 8 is a perspective view of another embodiment of a container for holding a plurality of rolled disposable absorbent articles;
FIG. 9 is a perspective view of still another embodiment of a container for holding a plurality of rolled disposable absorbent articles;
FIG. 10 is a bottom plan view of the container shown in FIG. 8; and
FIG. 11 is a flow diagram of a process for making a rolled disposable absorbent article.

### DETAILED DESCRIPTION OF THE INVENTION

As used herein, absorbent articles, shall mean disposable absorbent articles such as, pantiliners, sanitary napkins, interlabial devices, adult incontinent devices, breast pads, shoe insoles, bandages, and diapers. These articles are well known in the art. These articles typically have a fluid permeable body-facing side and fluid impermeable garment facing side. Additionally, such articles may include an absorbent core for retaining fluids therebetween.

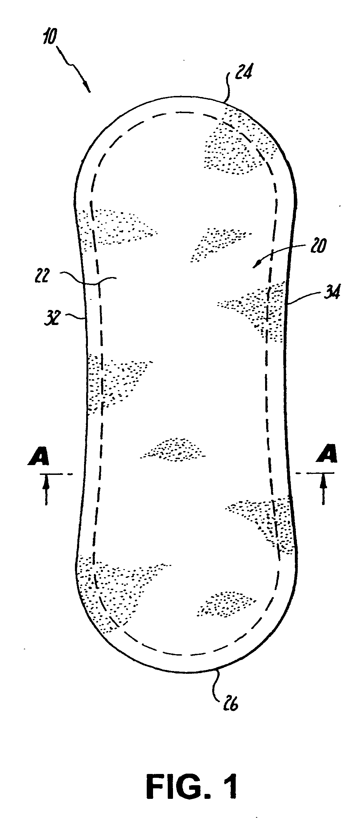

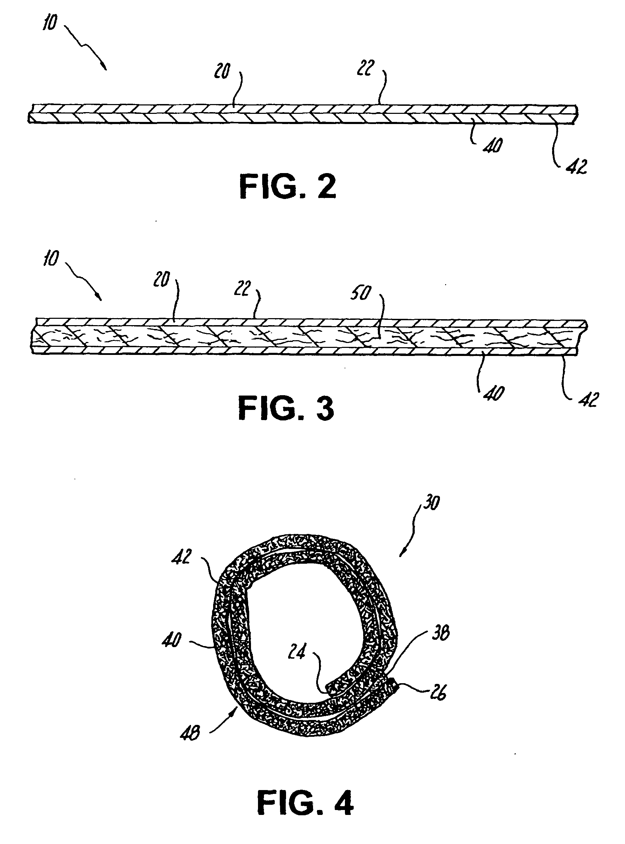

As seen in Figures 1-3, absorbent article 10 may assume any desired configuration in terms of shape and constructive particularities; but, generally speaking, it comprises a cover layer 20 having body facing surface 22, a backsheet 40 having a garment facing surface 42, and optionally, an absorbent core 50 made from absorbent material (shown in Figure 2). The absorbent article 10 has a first end 24, a second end 26, first longitudinal side 32 and second longitudinal side 34.

In one embodiment of this invention, the absorbent article 10 is put into a rolled configuration such that first end 24 forms the central portion of the resulting rolled disposable absorbent article 30. Second end 26 is on the outer surface 42 and the backsheet 40 forms the exterior surface 48 of the structure 30. In this embodiment, second end 26 is attached to exterior surface 48 by attachment means 38. Attachment means 38 may be any means such as thermobonding, chemical means, e.g., adhesive, and mechanical attachment means, e.g., clips, laces, ties, and interlocking devices, e.g., hook and loop, snaps, buttons, VELCRO (Velcro USA, Inc., Manchester, NH). This is shown in Figure 4. Alternately, the absorbent article may be rolled such that the body facing surface 22 of cover layer 20 forms the exterior surface 48.

In still another embodiment of the rolled disposable absorbent article 30 (not shown), the first longitudinal side 32 may form the central portion and the second longitudinal side 34 forms the exterior surface 48. As in the previous paragraph, the exterior surface 48 may be formed by the backsheet 40 or the cover 20.

Other rolling directions such as diagonally may also be used to form the rolled disposable absorbent article 30.

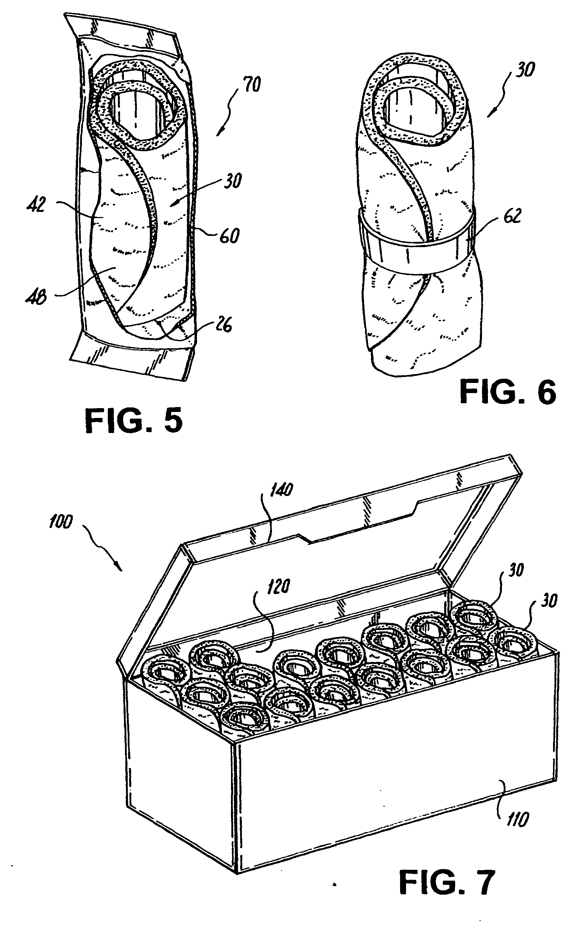

In the embodiment shown in Figures 4 and 5, it has been found that the tighter the rolling of the absorbent article, the smaller the diameter of the resulting rolled disposable absorbent article 30. In every embodiment of this invention, the central portion of the compact structure is formed by an end or side of the absorbent article 10. This allows the resulting rolled disposable absorbent article 30 to have a compact configuration and a small diameter.

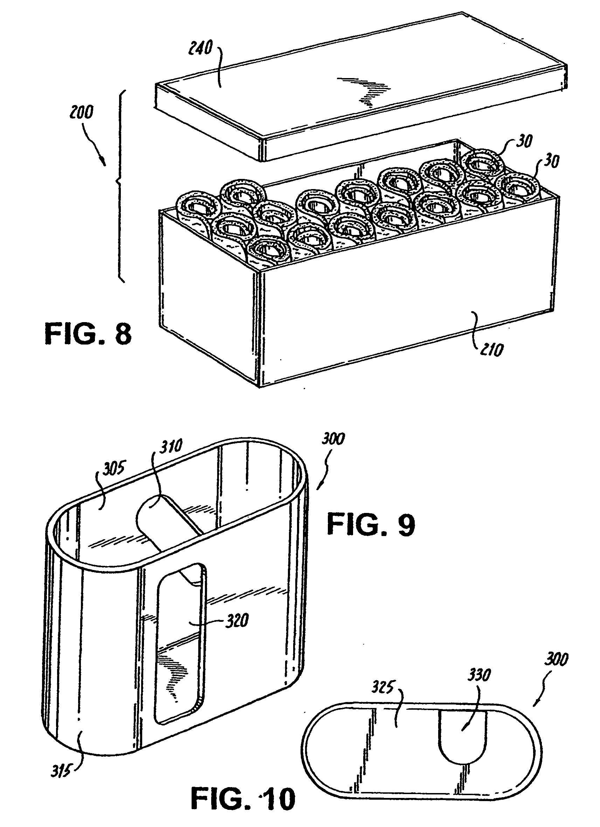

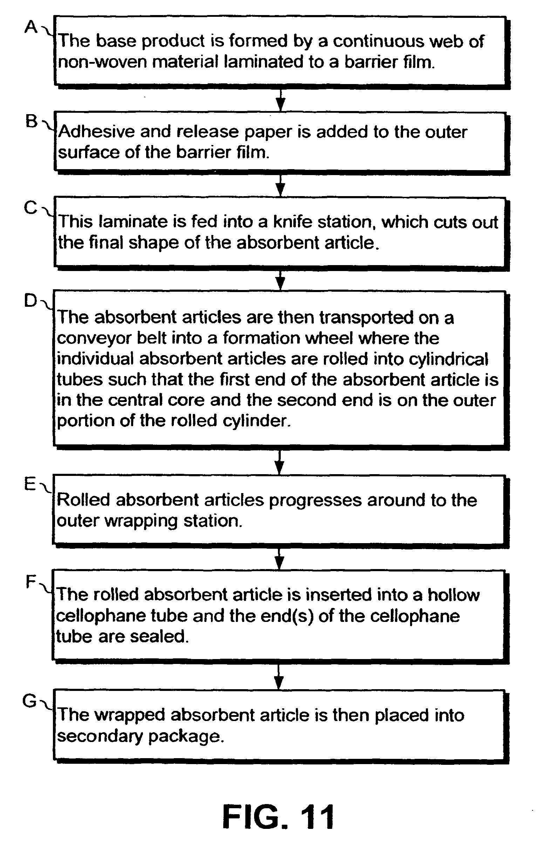

Once the absorbent article has been rolled into a rolled disposable absorbent article 30, it may be covered with an over-wrap 60 to form absorbent device 70 (shown in Figure 5). The over-wrap helps to prevent unrolling and contamination. In one embodiment, the compact structure is hermically sealed within a plastic wrap that protects the absorbent article from dust and dirt typically found in a user's purse. Alternately, the compact structure 30 may be surrounded by a ring 62 (shown in Figure 6) which helps keep the absorbent article in a rolled configuration. Multiples of absorbent device 70 may be further packaged in a container such as a flip-top box, a container having a removable top or plastic sack. Examples of such containers are shown in Figures 7-10. In one embodiment, the container 100 has a bottom receiving portion 110 which contains a plurality of individual rolled disposable absorbent articles 30. The top 140 is hingedly attached to the back wall 120 such that when the top 140 is flipped up into an open position (shown in Figure 7), an individual rolled structure 30 may be removed from the receiving portion 110. After the individual rolled disposable absorbent article 30 has been removed, the top 140 may be replaced in a closed position (not shown). In another embodiment, container 200 has a bottom receiving portion 210 that contains a plurality of individual rolled disposable absorbent articles 30. The top 240 is removable from the bottom receiving portion 210 (shown in Figure 8) and may be replaced once an individual rolled disposable absorbent article 30 is removed. In still another embodiment, container 300 is a unitary structure that has openings such that an individual rolled disposable absorbent article 30 (not shown) may be removed from the top 305, side 315 or bottom 325. Figures 10 and 11 show possible placements for the top opening 310, side opening 320 and bottom opening 330.

Additionally, the absorbent device may be packaged in a small purse-type container, typically made from plastic and which holds a few of the compact articles.

Any absorbent article as previously described may be used in this invention. In particular, in one embodiment of this invention, the absorbent article 10 may be drapeable, that is having a flexural resistance of about 35 g. or less as tested by the Modified Circular Bend Test, ASTM 4032-82 and as described in USSN 10/025299, Drapeable Absorbent Article, filed 12/19/01, the contents herewith incorporated in entirety by reference. In another embodiment, the absorbent article 10 has a cover 20, a backsheet 40, and a flexural resistance of less than 35 g.

It has been found that some absorbent articles such as those disclosed in USSN 10/025299, Drapeable Absorbent Article, hereby incorporated in entirety, are particularly well suited for rolling into the configuration as shown in Figures 3 and 4. For example, when an absorbent article made according to USSN 10/025299 having a cover 20, backsheet 40 and a flexural resistance of less than 35 g is rolled into a diameter of less than 15 mm and wrapped, the resulting product conforms to the underwear once it is unrolled. Once unrolled, the cover 20 retains no wrinkles or bend lines in the body facing surface and the flexural resistance is unchanged.

The absorbent article of the present invention is substantially devoid of any memory of being in a rolled configuration. That is, after being unrolled, the entire disposable absorbent article will lie substantially horizontal when placed on a horizontal surface.

Once the absorbent article is unrolled and the release paper removed, it can be placed onto the user's underwear. This particular embodiment conforms to the underwear and is not noticeable to the user.

### Cover

The absorbent article of the present invention includes a cover layer 20 overlaying the absorbent material. The exterior of the cover forms the body-facing surface 22 of the disposable absorbent article. As known by those skilled in the art, the cover layer 20 may be formed from any fluid pervious material that is generally compliant, soft feeling, and non-irritating to the user's skin and permits fluid to penetrate to the absorbent core, which retains the fluid. The cover layer 20 generally functions to transport fluid away from the wearer into the absorbent article. In this manner, fluid and moisture are removed from contacting the wearer, thus making the wearer feel dry and comfortable. In addition to transporting fluid, the cover layer 20 may also absorb and/or retain fluid as well.

The cover 20 can be made from any of the materials conventional for this type of use. Non-limiting examples of suitable materials that can be used as the cover layer 20 are woven and nonwoven fabrics formed fibers or filaments of cellulose, polyester, polypropylene, nylon, rayon fibers and mixtures thereof or the cover layer may be an apertured thermo-plastic film and formed films. Other materials used in making covers layer 20 include gauze or any known porous material with a suitable body contacting surface, including, but not limited to nonwoven webs, plastic nets, and the like. The cover layer 20 could also be made from a fibrous nonwoven composite of bicomponent fibers and pulp fluff.

Bicomponent fibers are known in the art and are composed of two polymers with different melting points. At least a portion of the outer surface of each bicomponent fiber has the lower melting polymer. The two polymers may be arranged such that a cross-section of the fiber shows the two polymers in a side-by-side array. Alternatively, the polymers may be positioned in a so-called sheath/core arrangement, in which a core of higher melting polymer is surrounded by a sheath of lower melting polymer. A useful bicomponent fiber is a 3.0 denier, 1.5" long staple fiber made of a polyester core and a high density polyethylene sheath. Similar fibers (polyethylene sheath and polypropylene core) are available as Danaklon ES-C or ES Bico (Danaklon A/S, Varde Denmark). Pulp fibers may be obtained as IP "'SUPERSOFT" ELM supplied by the International Paper Company (Memphis, Tennessee), "'RAYFLOC" XJ-HM E-Type Cellulosic Fluff Pulp, (ITT Rayonier), or Korsnas Vigorfluf-EN White (KorsncAs, Gavle, Finland).

The cover layer 20 may optionally be treated with surfactant to manipulate the hydrophobicity/hydrophilicty thereof to facilitate optimal fluid transport properties.
The fibers or other materials that make up the cover layer 20 should not collapse or lose their resiliency when subjected to body fluid. The fibers may be oriented by a carding process and thermally bonded via embossing. The fiber or filament can be single denier or multidenier.

The cover may be a single layer or be made from multiple layers. The thickness of the cover may vary from about 0.001 inch (0.025 mm) to about 0.200 inch (5.000 mm), depending on the material chosen. The weight of the body facing layer material preferably is between about 5 and about 150 gsm.

For example, any material with cloth-like features may be used for the body facing layer. Such material includes nonwoven, such as spunlace, woven, and knitted materials. In particular, spunlace material may be made from about 0 to about 100% rayon and from about 0 to about 100% polyester. The spunlace material may also be made from about 10 to about 65% rayon and from about 35 to about 90% polyester may be used. Optionally, the material used for the body-facing layer may include binders, such as thermoplastic binder fibers and latex binders.

In one embodiment, the cover is a single sheet of material having a width sufficient to form the body-facing surface of the absorbent article. In another embodiment, the cover has at least two layers.

The cover, whether a single layer or multiple layers, may also have absorbent capabilities, i.e., retains fluid. If a separate absorbent layer is used, the body facing layer may be longer and wider than the absorbent core or be of similar size as the absorbent core.

Generally, the optional cover layer 20 is a single sheet of material having a width sufficient to form the body-facing surface 22 of the absorbent article. The cover layer 20 may be longer and wider than the optional absorbent core.

The cover layer 20 may be embossed with shapes within a given area. For example, a series or a number of features, e.g., circles, triangles, squares, lines, honeycomb, diamond, floral, etc. are embossed over the entire length and width of the outer surface of web. Each embossed feature has a major and minor axis extending therethrough, the major axis length being greater or equal to the minor axis length. The embossed features may be in a repetitive pattern.

### Transfer layer

Optionally, the absorbent article of the present invention may include a transfer or distribution layer. The transfer layer or distribution layer, if present, is generally positioned beneath the cover 20 and the transfer layer usually directly contacts the absorbent core. If included, the transfer layer may be made of any known material that will take up fluid and then distribute and release it to an adjacent absorbent layer for storage. Transfer layers have a relatively open structure that allows for movement of fluid within the layer. Suitable materials for such transfer layers include fibrous webs, resilient foams, and the like.

The transfer layer provides a means of receiving body fluid from the fluid-pervious cover layer 20 and holding it until the absorbent core has an opportunity to absorb it. The transfer layer is, preferably, more dense than the cover layer 20 and has a larger proportion of smaller pores than does the cover layer 20. These attributes allow the transfer layer to contain body fluid and hold it away from the outer side of the cover layer 20, thereby preventing the fluid from re-wetting the cover layer 20 and its outer surface. However, the transfer layer is preferably not so dense as to prevent the passage of the fluid through the transfer layer and into the underlying absorbent core. Cover/Transfer Layer Laminate

A laminate formed from the cover and transfer layers may be used. In an embodiment, the composite layer includes an embossed pattern on the outer surface. For example, flowers and rails depicted in U.S. Des. Pat. No. 439,057 are embossed after the composite is formed, which results in an embossed pattern having flowers, rails, and squares

In one embodiment of the present invention, the cover and transfer layers are joined to form a laminate. This two layer structure is particularly useful in personal care products such as feminine sanitary protection products having body-contacting, facing or cover layers, such as, transfer or fluid handling layers, or as other components of personal care products. The laminates of the invention have been found to exhibit improved fluid-handling properties when used in disposable absorbent articles, such as, for instance, feminine sanitary protection products.

Apertured films are typically made from a starting film that is a thin, continuous, uninterrupted film of thermoplastic polymeric material. This film may be vapor permeable or vapor impermeable; it may be embossed or unembossed; it may be corona-discharge treated on one or both of its major surfaces or it may be free of such corona-discharge treatment; it may be treated with a surface active agent after the film is formed by coating, spraying, or printing the surface active agent onto the film, or the surface active agent may be incorporated as a blend into the thermoplastic polymeric material before the film is formed. The film may comprise any thermoplastic polymeric material including, but not limited to, polyolefins, such as, high density polyethylene, linear low density polyethylene, low density polyethylene, polypropylene; copolymers of olefins and vinyl monomers, such as, copolymers of ethylene and vinyl acetate or vinyl chloride; polyamides; polyesters; polyvinyl alcohol and copolymers of olefins and acrylate monomers, such as, copolymers of ethylene and ethyl acrylate and ethylenemethacrylate. Films having mixtures of two or more of such polymeric materials may also be used. The machine direction (MD) and cross direction (CD) elongation of the starting film to be apertured should be at least 100% as determined according to ASTM Test No. D-882 as performed on an Instron test apparatus with a jaw speed of 50 inches/minute (127 cm/minute). The thickness of the starting film is preferably uniform and may range from about 0.5 to about 5 mils or about 0.0005 inch (0.0013 cm) to about 0.005 inch (0.076 cm). Coextruded films can be used, as can films that have been modified, e.g., by treatment with a surface-active agent. The starting film can be made by any known technique, such as casting, extrusion, or blowing.

Aperturing methods are known in the art. Typically, a starting film is placed onto the surface of a patterned support member. The film is subjected to a high fluid pressure differential while on the support member. The pressure differential of the fluid, which may be liquid or gaseous, causes the film to assume the surface pattern of the patterned support member. Portions of the film overlying apertures in the support member are ruptured by the fluid pressure differential to create an apertured film. A method of forming an apertured fibrous film is described in detail in commonly owned US Pat. No. 5,827,597, which is incorporated herein by reference.

Apertured film can be made by any process or method know to those skilled in the art.

The two-layer structure may advantageously be used as a cover/transfer layer of an absorbent article, such as, a sanitary napkin, pantiliner, diaper, incontinence pad, or other similar product for absorbing exudates from the body, such as, menses, urine, feces, or sweat.

### Absorbent Structure

The absorbent core 50 of the present invention may contain any known absorbent materials including, but not limited to, absorbent fibers, such as, cellulose fibers, including, but not limited to wood pulp, regenerated cellulose fibers, e.g., rayon and cotton fibers, rayon fibers and the like; superabsorbent powders (SAP) like Sumitomo SA-70 or fibers (SAF), other naturally occurring absorbent materials, such as, sphagnum or peat moss; and other synthetic absorbent materials, such as, foams and the like. The absorbent core 50 may also be made from multiple components and contain a structure such as that disclosed in USSN 10/652,171, entitled DISPOSABLE ABSORBENT ARTICLES, filed 8/29/2003, which is herein incorporated by reference in its entirety. Additionally, the absorbent core 50 may include one or more of the following: binders, such as, thermoplastic and latex, odor-controlling compounds, e.g., perfumes, EDTA (ethylenediaminetetraacetic acid), anti-microbial agents, wetting agents, wetness indicator material, materials for administering or delivering medicaments, such as encapsulated medicaments, and materials for maintaining skin moisture, such as encapsulated moisturizers.

For example, the absorbent core 50 may be made from material such as a fluffy batt cut from a relatively loose web of non-woven fibers having a relatively high absorptive capacity. While the absorbent core can have any shape or silhouette, it usually has an asymmetric configuration. The absorbent core 50 may also be made from material such as a fibrous batt having an integral densified layer. In such a case, if a backsheet is desired, the absorbent core is positioned on the backsheet of the absorbent article so that the densified layer adjoins the backsheet. The densified layer has relatively higher wettability and liquid retentivity than the rest of the aforesaid batt and usually is formed by slightly moistening one surface of the batt and thereafter compressing the moistened surface. The absorbent core 50 may also be formed from multiple layers, each having a different density such that the uppermost layer (closest to the body) is less dense than the outer (closest to the garment).

Additionally, the absorbent core 50 may be formed of absorbent material made from an oflline-formed, homogeneously mixed, air-laid layer, roll good laminate or any other offline-formed absorbent composite.

The absorbent core may include only materials, such as, a hot melt adhesive containing fluid absorbing polymers. One example of such a material is disclosed in EP 1 013 291 A1, the disclosure of which is herein incorporated in entirety by reference.

Additionally, additives may be incorporated into the absorbent core, such as, surfactants, SAP, and SAF. These additives may provide additional benefits such as enhanced fluid penetration and increased fluid absorption. For example, in one embodiment, the absorbent layer is made of absorbent material that is made from a layer of pulp. In another embodiment, SAP is mixed with the pulp to form an absorbent composite. This composite may be condensed to form a dense, thin layer. One example of such a material is Novathin® available from Rayonier, Jesup, GA.

SAP are particles that are capable of absorbing many times, at least 10, more preferably 15, and still more preferably over 15, their weight in exudate, under a pressure of 0.5 psi. It should be noted that, in the context of the present invention, there is no restriction that the superabsorbent particles actually be particulate. This expression is intended to cover superabsorbent fibers, and other superabsorbent materials, whatever their form and shape. These superabsorbent particles generally fall into three classes, namely starch graft copolymers, cross-linked carboxymethylcellulose derivates and modified hydrophilic polyacrylates. Examples of such absorbent polymers are hydrolyzed starch-acrylonitrile copolymer graft copolymer, a neutralized starch-acrylic acid graft copolymer, a saponified acrylic acid ester-vinyl acetate copolymer, a hydrolyzed acrylonitrile copolymer or acrylamide copolymer, a modified cross-linked polyvinyl alcohol, a neutralized self cross-linking polyacrylic acid, a cross-linked polyacrylate salt, carboxylated cellulose, and a neutralized cross-linked isobutylene-malasic anhydride copolymer. In one embodiment of the invention, the superabsorbent particle is a cross-linked polyacrylate salt.

### Barrier Layer

The barrier layer, also called backsheet 40, may be located adjacent to the cover as shown in Figure 2. The barrier may also be located adjacent to absorbent core 50 if one is used (shown Figure 3) and also to the cover 20, especially if the absorbent core is smaller than the cover and barrier. The barrier layer 40 of the present invention is a body fluid impervious material, which is at least substantially impermeable to liquids. Its exterior forms the garment-facing surface of the absorbent article. The backsheet 40 may be any thin, flexible, body-fluid impermeable material, such as, but not limited to, a polymeric film, e.g., polyethylene, polypropylene, or cellophane, or a normally fluid pervious material that has been treated to be impervious, such as impregnated fluid repellent paper or non-woven material, including nonwoven fabric material, or a flexible foam, such as polyurethane or cross-linked polyethylene. Optionally, the backsheet 40 may be breathable, i.e., permits vapor to transpire. Known materials for this purpose include nonwoven materials, monolithic and microporous films in which microporosity is created by, inter alia, stretching an oriented film. Single or multiple layers of permeable films, fabrics, melt-blown materials, and combinations thereof that provide a tortuous path, and/or whose surface characteristics provide a liquid surface repellent to the penetration of liquids may also be used to provide a breathable backsheet.

### Bonding Methods

The layers of the absorbent article may be, but not necessarily, bonded, e.g., glued or adhered, to the adjacent layer. For example, the underside of the cover 20 may be adhered to the topside of the absorbent core 50. The underside of the absorbent core 50 may be adhered to the topside of the barrier layer 40. Any methods known in the art, such as, fusion bonding, adhesive attachment, or by any other securement means can be used to secure the individual layers together to form the final absorbent article. Included within such methods are coembossing, thermobonding, mechanical bonding, and the like. Fusion bonding includes heat bonding, ultrasonic bonding, and the like.

Adhesive is typically used to attach the layers into a single absorbent article. For example, in one embodiment, the body facing cover 10 is attached to the barrier layer 50 with adhesive HL 1491 available from H.B Fuller and Company (St. Paul, MN). The adhesive may be applied in any method.

Adhesive may include pressure sensitive adhesive that is applied as strips, swirls, or waves, and the like. As used herein, the term pressure-sensitive adhesive refers to any releasable adhesive or releasable tenacious means. Suitable adhesive compositions, include, for example, water-based pressure-sensitive adhesives such as acrylate adhesives. Alternatively, the adhesive composition may include adhesives based on the following: emulsion or solvent-borne adhesives of natural or synthetic polyisoprene, styrene-butadiene, or polyacrylate, vinyl acetate copolymer or combinations thereof; hot melt adhesives based on suitable block copoylmers - suitable block copolymers for use in the invention include linear or radial co-polymer structures having the formula (A-B)x wherein block A is a polyvinylarene block, block B is a poly(monoalkenyl) block, x denotes the number of polymeric arms, and wherein x is an integer greater than or equal to one. Suitable block A polyvinylarenes include, but are not limited to Polystyrene, Polyalpha-methylstyrene, Polyvinyltoluene, and combinations thereof. Suitable Block B poly(monoalkenyl) blocks include, but are not limited to conjugated diene elastomers such as for example polybutadiene or polyisoprene or hydrogenated elastomers such as ethylene butylene or ethylene propylene or polyisobutylene, or combinations thereof. Commercial examples of these types of block copolymers include Kraton™ elastomers from Shell Chemical Company, Vector™ elastomers from Dexco, Solprene™ from Enichem Elastomers and Stereon™ from Firestone Tire & Rubber Co.; hot melt adhesive based on olefin polymers and copolymers where in the olefin polymer is a terpolymer of ethylene and a comonomers, such as vinyl acetate, acrylic acid, methacrylic acid, ethyl acrylate, methyl acrylate, n-butyl acrylate vinyl silane or maleic anhydride. Commercial examples of these types of polymers include Ateva( polymers from AT plastics), Nucrel( polymers from DuPont), Escor (from Exxon Chemical).
In one embodiment, the cover and core are co-embossed into an absorbent structure. Depressions formed from the co-embossing allow the absorbent article to be rolled into a compact structure without the cover surface wrinkling. Upon unrolling and removal of the release paper, the absorbent article does not retain "memory" of being rolled. In other words, the absorbent article lays flat or conforms to the article to which it is placed on, in this instance, the crotch portion of a garment such as underwear.

The absorbent article of the present invention may be applied to the crotch of a garment by placing the garment-facing surface against the inside surface of the crotch of the garment. Various methods of attaching absorbent articles may be used. For example, chemical means, e.g., adhesive, and mechanical attachment means, e.g., clips, laces, ties, and interlocking devices, e.g., snaps, buttons, VELCRO (Velcro USA, Inc., Manchester, NH), zipper, and the like are examples of the various options available to the artisan.

Adhesive may be applied to the garment-facing side of the absorbent article. The positioning adhesive may be any adhesive known in the art. As a non-limiting example, pressure sensitive adhesive strips, swirls, or waves may be applied to help maintain the absorbent article in place. As used herein, the term pressure-sensitive adhesive refers to any releasable adhesive, or releasable tenacious means. Suitable adhesive compositions, include, for example, water-based pressure-sensitive adhesives, such as acrylate adhesives. Alternatively, the adhesive composition may include rapid setting thermoplastic "hot melt," rubber adhesives, two-sided adhesive tape, and the like.

Where positioning adhesive is used on the garment-facing side of the barrier layer 40, a release strip may be applied to protect the adhesive on the absorbent article prior to attaching the absorbent article to the crotch. The release strip can be formed from any suitable sheet-like material that adheres with sufficient tenacity to the adhesive to remain in place prior to use but which can be readily removed when the absorbent article is to be used. Optionally, a coating may be applied to release strip to improve the ease of removabilty of the release strip from the adhesive. Any coating capable of achieving this result may be used, e.g., silicone. Because typical release strips have memory properties, it is contemplated that while a release strip can be used with the rolled disposable article of the present invention, the memory properties of the disposable absorbent article of the present invention are separate and distinct from any memory properties of the release paper.

### Wings

Wings, also called, among other things, flaps or tabs, may also be part of the absorbent article of the present invention. Wings and their use in sanitary protection articles are described in U.S. Patent. No. 4,687,478 to Van Tilburg; U.S. Patent No. 4,589,876 also to Van Tilburg, U.S. Patent No. 4,900,320 to McCoy, and U.S. Patent No. 4,608,047 to Mattingly. The disclosures of these patents are incorporated herein by reference in their entirety.

As disclosed in the above documents, wings are, generally speaking, flexible and configured to be folded over the edges of the underwear so that the wings are disposed between the edges of the underwear.

In addition, there may be one or a plurality of wings incorporated into the present invention. Where opposed wings are present along the longitudinal edges of the absorbent article of the present invention, such wings may be directly opposed or may be offset from the wings located along the opposite longitudinal edge of the absorbent article.

When present, the wings may be folded inwardly or outwardly prior to rolling the disposable absorbent article.

### Overwrap

The rolled absorbent article can be maintained in a rolled configuration using a wrapper, a ring, a hook and loop system, adhesives and mixtures thereof. For example, a wrapper made of standard cellophane or polypropylene films and including any heat sealable material may be used.

The overwrap can also be embossed (including microembossing), electronically altered (including corona discharge treatments), coated (including vapor deposition, and sputtering) and the like.

In addition, slip agents such as fluid lubricants or solid layers with a reduced coefficient of friction may be applied to the overwrap package at any appropriate portion of the manufacturing process.

Additionally, the wrapper may have a tear strip located near the middle of the product, and one or more portions of the package are then slid off the ends.

### Miscellaneous

Any or all of the cover, absorbent layer, transfer layer, backsheet layer, and adhesive layers may be colored and/or transparent. Such coloring includes, but is not limited to, white, black, red, yellow, blue, orange, green, violet, and mixtures thereof. Color may be imparted according the present invention through dying, pigmentation, and printing. Colorants used according the present invention include dyes and inorganic and organic pigments. The dyes include, but are not limited to, anthraquinone dyes (Solvent Red 111, Disperse Violet 1, Solvent Blue 56, and Solvent Green 3), Xanthene dyes (Solvent Green 4, Acid Red 52, Basic Red 1, and Solvent Orange 63), azine dyes (Jet black), and the like.

Inorganic pigments include, but are not limited to, titanium dioxide (white), carbon black (black), iron oxides (red, yellow, and brown), chromium oxide (green), ferric ammonium ferrocyanide (blue), and the like.

Organic pigments include, but are not limited to diarylide yellow AAOA (Pigment Yellow 12), diarylide yellow AAOT (Pigment Yellow 14), phthalocyanine blue (Pigment Blue 15), lithol red (Pigment Red 49:1), Red Lake C (Pigment Red), and the like.

Also contemplated herein include asymmetrical and symmetrical articles having parallel longitudinal edges, dog bone- or peanut-shaped, circular, oval and the like.

The silhouette of the disposable absorbent article of the present invention may be configured to be used with conventional underwear or may be configured to conform to thong garments. As used herein, the term thong includes, but is not limited to, thong underwear, thong swimming suit bottom, G-strings, Rio cut underwear, Rio cut swimming suit bottom, Brazilian cut underwear, Brazilian cut swimming suit bottom, and any other garment that exposes the buttocks, having a narrow strip of fabric or a cord that passes between the thighs supported by a waistband, a waist cord, belt or the garment itself. The absorbent article may include other known materials, layers, and additives, such as, foam, net-like material, perfumes, medicaments or pharmaceutical agents, moisturizers, odor control agents, and the like. The absorbent article can optionally be embossed with decorative designs.

The absorbent article 10 may be made from any of the processes known to one of ordinary skill in the art. For example, a continuous length of cover material may be laminated to a continuous length of absorbent material. This laminate may then be laminated to a backsheet to form an absorbent article. The absorbent article can then be rolled up and over-wrapped to form a single unitary or individual absorbent article. In another embodiment, the cover is laminated to the backsheet and then rolled up and over-wrapped.

In one embodiment, as shown in Figure 4, an absorbent article 10 is processed in the following manner: a continuous web of spunlace nonwoven material made from about 70% rayon and 30% polyester is laminated to a liquid impervious barrier film. Adhesive and release paper is added to the outer surface of the barrier film. This laminate is fed into a knife station, which cuts out the final shape of the absorbent article. In this embodiment, the resultant shape is a peanut-like shape as seen in US Des. Pat. No. 439,057. The absorbent articles are then transported on a conveyor belt into a formation wheel where the individual absorbent articles are rolled into cylindrical tubes such that the first end of the absorbent article is in the central core and the second end is on the outer portion of the rolled cylinder. In one embodiment, the absorbent article is fed from a conveyor belt into a formation wheel having a series of posts; each of the absorbent articles encircle a post as the formation wheel progresses around to the outer wrapping station. The formation wheel has a diameter of about 650mm with some space between the posts. The formation wheel could have between 1 to 40 posts with 40 being preferred, and each post has a diameter of about 10 to about 30mm.

In another embodiment the rolling step is accomplished by use of a fork having two fingers. The first end of the absorbent article is captured between the two fingers and the fork is turned such that the absorbent article is rolled around the fingers. After the absorbent article is completely rolled up, the fork is removed and the absorbent article progresses to the wrapping station. The end of the absorbent article forms the central portion of the rolled absorbent article. The resulting rolled disposable absorbent article has about 15 ± 5mm.

At the wrapping station, cellophane is processed such that a hollow tube with at least one open end is formed. The cellophane tube is slightly larger than the rolled absorbent article. The rolled absorbent article is inserted into the hollow cellophane tube and the end(s) of the cellophane tube are sealed. The cellophane tube may include a tear strip such as those disclosed in U.S Pat. No 4170305 to Hull.

The wrapped absorbent article is then placed into secondary package, e.g., box, pouch, container, and the like..

The entire contents of all patents and patent applications listed or described above are incorporated herein by reference.

From the foregoing description, one skilled in the art can ascertain the essential characteristics of this invention, and without departing from the spirit and scope thereof, can make various changes and modifications. Embodiments set forth by way of illustration are not intended as limitations on the variations possible in practicing the present invention.

Although the invention is illustrated and described above with reference to specific embodiments, the invention is not intended to be limited to the details shown. Rather, various modifications may be made in the details within the scope and range of equivalents of the claims and without departing from the invention. It is expressly intended, for example, that all ranges broadly recited in this document include within their scope all narrower ranges that fall within the broader ranges.

## Claims

1. An individually rolled disposable absorbent article comprising a disposable absorbent article comprising
a) a silhouette comprising two opposed end portions and two opposed longitudinal edges connecting the two opposed end portions and
b) a plurality of layers comprising a cover and a backsheet, wherein the disposable absorbent article is rolled from one end portion to the opposed end portion to form the rolled disposable absorbent article and upon unrolling the rolled disposable absorbent article, the absorbent article maintains substantially no memory of its prior rolled configuration.

2. An individually rolled disposable absorbent article of claim 1, wherein the backsheet forms an outer surface of the absorbent article in the rolled configuration.

3. An individually rolled disposable absorbent article of claim 1, wherein the cover forms an outer surface of the absorbent article in the rolled configuration.

4. An individually rolled disposable absorbent article of claim 1 further comprising an absorbent core.

5. An individually rolled disposable absorbent article of claim 1 wherein the absorbent article has an MCB of less than about 35.

6. An individually rolled disposable absorbent article of claim 1 or 5, wherein the cover comprises a material selected from the group consisting of a spunlace, a woven, and a knit.

7. An individually rolled disposable absorbent article of claim 6, wherein the cover consists essentially of a spunlace material that, preferably, comprises polyester and rayon.

8. An individually rolled disposable article of any of the previous claims, wherein the rolled disposable article is maintained in a rolled position using a device selected from the group consisting of a wrapper, a ring, a hook and loop, an adhesive, and mixtures thereof, and, preferably, a polymer wrapper that encases the rolled disposable absorbent article.

9. An individually rolled disposable absorbent article according to claim 1 consisting essentially of components a) and b), wherein the cover is a spunlace cover and the backsheet is a microporous film backsheet.

10. A package for holding a plurality of individually rolled disposable absorbent articles comprising
a container having a top, base and at least one side connecting the top to the base,
means for removing an individually rolled disposable absorbent article from the container, and
a plurality of individually rolled disposable absorbent articles.

11. A package of claim 10, wherein each individually rolled disposable absorbent article is maintained in a rolled configuration using a device selected from a wrapper, a ring, a hook and loop, an adhesive, and mixtures thereof and, preferably, a polymer wrapper that encases the rolled disposable absorbent article.

12. A package of claim 10, wherein each individually rolled absorbent article comprises a disposable absorbent article as claimed in any of claims I to 9.

13. A package of any of claims 10 to 12, wherein the means for removing each individually rolled disposable absorbent article is selected from the group consisting of a removable top, a hingedly attached top, a resealable top, an opening in the side, an opening in the bottom, and an opening in the top.

14. A method of providing a package containing at least one individually rolled disposable absorbent articles, the method comprising:
(a) providing a cover material,
(b) providing a backsheet,
(c) securing the cover to the backsheet to provide a disposable absorbent article,
(d) rolling the disposable absorbent article to form an individually rolled disposable absorbent article,
(e) applying means to maintain the individually rolled disposable absorbent article in a rolled configuration, and
(f) placing the individually rolled disposable absorbent article in a package.

15. A method of claim 14, wherein the means to maintain the individually rolled disposable absorbent article in a rolled configuration is selected from the group consisting of a wrapper, a ring, a hook and loop, an adhesive, and mixtures thereof and, preferably, wherein the wrapper is plastic and is hermetically sealed.
